# EUROPEAN PATENT APPLICATION

(11) **EP 4 491 028 A1**
(43) Date of publication of application: **15.01.2025**
(21) Application number: 24188008.7
(22) Date of filing: 11.07.2024
(51) Int. Cl.: A23G 3/48

(54) **BITTER TEA CANDY MANUFACTURING METHOD, AND BITTER TEA CANDY**

(30) Priority: 13.07.2023 TW 112126187
(71) Applicant: Hsu-Lin, Wu, Tainan City 712 (TW)
(72) Inventor: Hsu-Lin, Wu, Tainan City 712 (TW)
(74) Representative: Jannig & Repkow Patentanwälte PartG mbB

(57) **Abstract**

A bitter tea candy manufacturing method includes: using fish mint, cut-leaf ground berry, Indian spherical turmeric, flemingia macrophylla, dried ginger, Indian peppermint, wakame, Indian echinacea and spreading hedyotis for a first decoction to obtain a green herb extract liquid; using the green herb residues remained for a second decoction to obtain a green herb extract liquid; re-extracting the first- and second-decoction extracted liquids to obtain a green herb extract concentrated liquid; adding the second-decoction noni fruit powder, maca powder, acanthopanax powder, rhodiola powder and naturally extracted strip menthol crystal grain and a small amount of excipients into the green herb extract concentrated liquid; rapidly freezing and grounding the mixed liquid to obtain a green herb extract powder; mixing the green herb extract powder with syrup, Vitamin C, and food grade menthol crystal powder to produce a candy; shaping the candy; and packaging the candy with an aluminium foil.

## Description

### BACKGROUND

### Technical Field

The present disclosure relates to a method, more particularly relates to a bitter tea candy manufacturing method that includes the steps of obtaining a green herb extract liquid through a second decoction, extracting the extract liquid to obtain a green herb extract concentrated liquid, adding five second-decoction plant powder granules and a small amount of excipients which are mixed and stirred to obtain a mixed liquid, freezing the mixed liquid and grounding it to obtain a green herb extract powder, and finally mixing the green herb extract powder with liquid paste syrup, Vitamin C and food grade menthol crystal powder to produce a candy which is set still, shaped, and packaged into a bitter tea candy product; and a bitter tea candy manufactured by the bitter tea candy manufacturing method, and the bitter tea candy includes a green herb extract powder of a concentrated, frozen and dried green herb, which is mixed with granulated sugar, maltose, Vitamin C and food grade menthol crystal powder, and the bitter tea candy is formed into a granular shape and wrapped with aluminium foil.

### Description of Related Art

In general, the manufacturing of throat lozenges for the relief of throat discomfort is very complicated, not only involving the difficulties of obtaining the required raw materials, but also having the difficulties to include total alkaloids, mannitol, calcium, iron, magnesium, selenium and total polyphenol in the throat lozenges, and produce appropriate bitterness through alkaloids. In addition, the conventional products are usually made of non-hard sugar and formed into triangular soft candies and coated with sugar granules, so that the conventional throat lozenges cannot soothe the throat discomfort by slowly releasing the ingredients of the lozenges in mouth, thus significantly reducing the utility of the throat lozenges. This is what the persons skilled in the art and consumers are eager to break through.

### SUMMARY

In view of the aforementioned deficiencies of the related art, it is a primary objective of the present disclosure to overcome the deficiencies of the related art by providing a bitter tea candy manufacturing method and a bitter tea candy. The bitter tea candy manufacturing method includes the steps of: preparing nine green herbs which are used for a first decoction to obtain a green herb extract liquid, performing a second decoction of the green herb residues remained after the first decoction to obtain a green herb extract liquid, re-extracting the first- and second-decoction extracted liquid to obtain a green herb extract concentrated liquid, adding, mixing and stirring five second-decoction plant powder granules and a small amount of excipients in the green herb extract concentrated liquid to obtain a mixed liquid, freezing and grounding the mixed liquid to obtain a green herb extract powder, adding Vitamin C and food grade menthol crystal powder and mixing the green herb extract powder with a liquid paste syrup to produce a candy, setting the candy still and shaping the candy, and packaging the candy.

A secondary objective of this disclosure is to provide a bitter tea candy manufacturing method and a bitter tea candy, and the bitter tea candy is manufactured by the aforementioned bitter tea candy manufacturing method, and the bitter tea candy includes a plurality of green herb extract powders formed by concentrating, freezing and drying a green herb, and mixed with granulated sugar, maltose, Vitamin C and food grade menthol crystal powder to manufacture the bitter tea candy in a granule shape; and the bitter tea candy is wrapped with an aluminium foil.

A further objective of this disclosure is to provide a bitter tea candy manufacturing method and a bitter tea candy manufactured by the same manufacturing method and available in form of hard candy or oral tablet (hard mouth candy) with a green herb alkaloid that produces an appropriate bitterness and a menthol cooling taste.

Another objective of this disclosure is to provide a bitter tea candy manufacturing method and a bitter tea candy that effectively improve the convenience of use and carrying.

The objective of this disclosure is to provide a throat lozenge for the relief of throat discomfort and overcome the complexity of its manufacture and the difficult access of its raw materials. Since the conventional throat lozenge cannot contain total alkaloid, mannitol, calcium, iron, magnesium, selenium and total polyphenol, while producing appropriate bitterness through alkaloids, and the conventional throat lozenge is usually a non-hard candy in form of a triangular soft candy, and the outer surface of the soft candy is coated with sticky sugar granules, therefore it is unable to slowly relieve the ingredients of the mouth candy in order to soothe the discomfort of the throat of human being, greatly reducing the practicality and effect of the throat lozenge.

To achieve the aforementioned objective and overcome the deficiencies of the related art, this disclosure provides a bitter tea candy manufacturing method, and the bitter tea candy manufacturing method includes the steps of:
(1) preparing nine green herbs for a first decoction to obtain a green herb extract liquid, i.e., preparing approximately 400 Kg of the first-time nine natural botanical edible green herbs, putting them into two separate metal barrels after cleaning and fine cutting, adding approximately 200 Kg of the green herbs and approximately 300 liters of water in each barrel, heating to decoct for approximately 6-8 hours to concentrate the green herb extract liquid to approximately 100 liters for each metal barrel, where the two metal barrels contain approximately 200 liters; the green herb is a dried edible plant material containing fish mint (scientific name: Houttuynia Cordata Thunb), cut-leaf ground berry (scientific name: Physalis Angulaa L), Indian spherical turmeric (scientific name: Curcuma Longa), flemingia macrophylla (common name: Root of Moghania; scientific name: Monghania Macrophylla (Willd) O Kuntze), dried ginger (scientific name: Zingiber Officinale), Indian peppermint (common name: Spanish thyme or Mexican mint; scientific name: Coleus Ambonicus Lour), wakame (scientific name Undaria Pinnatifida), Indian Echinacea (common name: Chuanxinlian; scientific name: Andrographis Paniculata (Burmf) Nees) and Spreading Hedyotis (common name: Snake Needle Grass; scientific name: Hedyotis Diffusa Willd);
(2) using the green herb residues remained after the first decoction for a second decoction to obtain a green herb extract liquid, i.e., decocting, concentrating and extracting the green herb residues remained after the first decoction of the nine natural botanical edible green herbs to obtain the green herb extract liquid in the step (S1), and adding approximately 250-300 liters of water for the second time and putting them into the metal barrels to heat, decoct and concentrate for approximately 6-8 hours to obtain approximately 200 liters of a green herb extract liquid;
(3) re-extracting the first- and second-decoction extracted liquid to obtain a green herb extract concentrated liquid, i.e., re-extracting the first- and second-decoction green herb extract liquid to obtain approximately 400 liters of the green herb extract liquid, heating and concentrating the green herb extract liquid to approximately 170 liters of a high-concentration alkaloid green herb extract concentrated liquid;
(4) adding, mixing and stirring five second-decoction plant powder granules and a small amount of excipients in the green herb extract concentrated liquid to obtain a mixed liquid, i.e., adding five second-decoction plant powder granules comprising noni fruit powder, maca powder, acanthopanax powder, rhodiola powder and naturally extracted strip menthol crystal grain and corn starch and maltodextrin as excipients to approximately 170 liters of the green herb extract concentrated liquid of the nine botanical green herbs of the step (S3) at a temperature approximately 60°C-80°C, where there are fourteen types of plants, and setting at room temperature to obtain approximately 170 liters of a mixed liquid;
(5) rapidly freezing and grounding the mixed liquid to obtain a green herb extract powder, i.e., putting approximately 170 liters of the mixed liquid of the step (S4) into a freezing equipment at -20°C to freeze the mixed liquid to a flake solid, and grounding the flake solid to obtain approximately 9.5 Kg of a green herb extract powder;
(6) mixing a liquid paste syrup with the green herb extract powder and then adding Vitamin C and food grade menthol crystal powder to produce a candy, i.e., mixing and heating approximately 9.5 Kg of the green herb extract powder of the step (S5) with approximately 390 Kg of granulated sugar and maltose to 60°C to form a liquid paste syrup, which is used as a candy base agent, and then adding, mixing and uniformly stirring Vitamin C and food grade menthol crystal powder in the liquid paste syrup, maintaining a temperature of 40-45°C, and extruding into a machine tube extrusion mold to produce a candy, wherein each piece of the candy weighs approximately 4.8±0.1 grams;
(7) setting and shaping the candy, i.e., setting the candy still for two days and cooling the candy to room temperature; and
(8) packaging the candy, i.e., wrapping each piece of candy of step (S7) with an aluminium foil to produce a product.

To achieve the aforementioned objective and overcome the deficiencies of the related art, this disclosure also provides a bitter tea candy manufactured by the bitter tea candy manufacturing method, and the bitter tea candy contains a plurality of green herb extract powders formed by concentrating, freezing and drying a plurality of green herbs, and the plurality of green herb extract powders, and mixed with granulated sugar, maltose, Vitamin C and food grade menthol crystal powder to produce the bitter tea candy in a granule shape, and each piece of the bitter tea candy weighs approximately 4.8±0.1 grams.

Wherein, the bitter tea candy of this disclosure is wrapped with an aluminium foil.

Wherein, the green herb extract powder of the bitter tea candy of this disclosure contains fish mint (scientific name: Houttuynia Cordata Thunb), cut-leaf ground berry (scientific name: Physalis Angulaa L), Indian spherical turmeric (scientific name: Curcuma Longa), flemingia macrophylla (common name: Root of Moghania; scientific name: Monghania Macrophylla (Willd) O Kuntze), dried ginger (scientific name: Zingiber Officinale), Indian peppermint (common name: Mexican mint; scientific name: Coleus Ambonicus Lour), wakame (scientific name: Undaria Pinnatifida), Indian Echinacea (common name: Chuanxinlian; scientific name: Andrographis Paniculata (Burmf) Nees), Spreading Hedyotis (common name: Snake Needle Grass; scientific name: Hedyotis Diffusa Willd), noni fruit powder (scientific name: Morinda Citrifolia), maca powder (scientific name: Lepidm Meyenli Walp, alias: Lepidium Peruvianum), acanthopanax powder (scientific name: Eleuthero Coccus Sentticosus), rhodiola powder (scientific name: Rhodiola Rosea L) and naturally extracted strip menthol crystal grain (scientific name: Menthol) and corn starch and maltodextrin as excipients.

Wherein, each gram of the bitter tea candy of this disclosure contains approximately 2.45±0.1mg/g of total alkaloid, approximately 4.13±0.1mg/g of mannitol, approximately 1.41±0.1mg/100g of calcium, approximately 0.6±0.1mg/100g of iron, approximately 26.2±0.1mg/100g of magnesium, approximately 0.1±0.01mg/100g of selenium, approximately 1.22±0.1mg/g of total polyphenol, and approximately 10±0.1mg/g of menthol.

Wherein, the bitter tea candy of this disclosure is in a form including but not limited to a hard candy or an oral tablet with a pH value of 5.32±0.02.

Compared with the effect of the related art, the bitter tea candy manufacturing method of this disclosure includes the steps of: preparing and using nine green herbs for a first decoction to obtain a green herb extract liquid; using the green herb residues remained after the first decoction for a second decoction to obtain a green herb extract liquid; re-extracting the first- and second-decoction extracted liquids to obtain a green herb extract concentrated liquid; adding, mixing and stirring five second-decoction plant powder granules and a small amount of excipients to the green herb extract concentrated liquid; rapidly freezing and grounding the mixed liquid to obtain a green herb extract powder; mixing the green herb extract powder with liquid paste syrup, Vitamin C and food grade menthol crystal powder to produce a candy; setting and shaping the candy; and packaging and wrapping the candy with an aluminium foil. The bitter tea candy is manufactured by the aforementioned bitter tea candy manufactured and contains a plurality of green herb extract powders formed by concentrating, freezing and drying a plurality of green herbs, and the plurality of green herb extract powders is mixed with granulated sugar, maltose, Vitamin C and food grade menthol crystal powder to produce a bitter tea candy in a granule shape;
the bitter tea candy is wrapped with an aluminium foil, thus achieving a consistent manufacturing process, and the bitter tea candy in form of a hard candy or an oral tablet (hard mouth candy) has the appropriate bitterness of herb alkaloids and the cooling taste of menthol, and this disclosure significantly improves the convenience of use and carrying and increases the practical utilization in the industry. In addition, this disclosure is novel and inventive.

### BRIEF DESCRIPTION OF THE DRAWINGS

FIG. 1 is a block diagram showing the steps of flow chart of a bitter tea candy manufacturing method of this disclosure;
FIG. 2 is a perspective view showing a bitter tea candy of this disclosure; and
FIG. 3 is a perspective view showing a bitter tea candy of this disclosure wrapped with an aluminium foil on its outer side.

### DESCRIPTION OF THE EMBODIMENTS

This disclosure will now be described in more details with reference to the accompanying drawings that show various embodiments of this disclosure. It is intended that the embodiments and drawings disclosed herein are to be considered illustrative rather than restrictive, and also noteworthy that the drawings are not necessary drawn according to the real proportion and precise configuration and these attached drawings should not limit the scope of the patent of this disclosure in actual implementation.

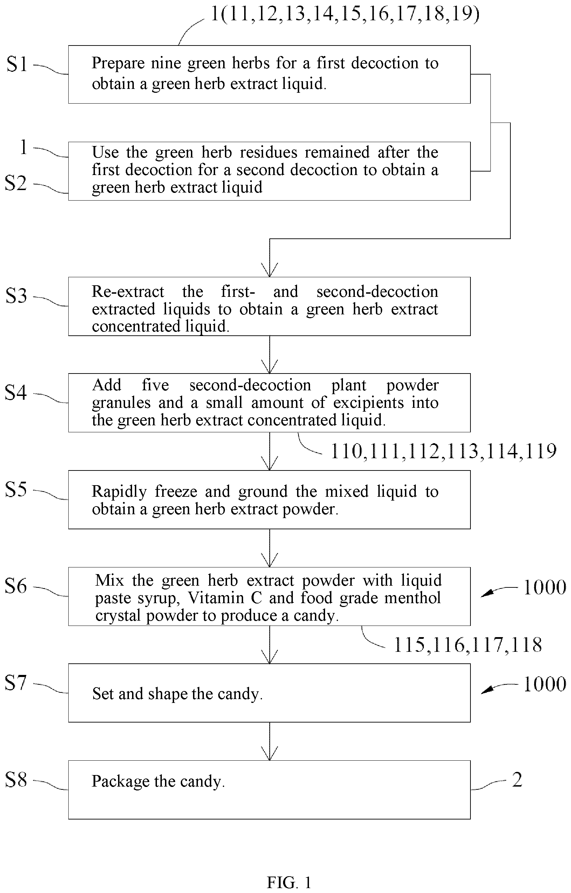

With reference to FIG. 1 for a block diagram showing the steps of flow chart of a bitter tea candy manufacturing method of this disclosure, the bitter tea candy manufacturing method includes the steps of: (S1) preparing nine green herbs for a first decoction to obtain a green herb extract liquid; (S2) using the green herb residues remained after the first decoction for a second decoction to obtain a green herb extract liquid; (S3) re-extracting the first- and second-decoction extracted liquids to obtain a green herb extract concentrated liquid; (S4) adding, mixing and stirring five second-decoction plant powder granules and a small amount of excipients in the green herb extract concentrated liquid to obtain a mixed liquid, (S5) rapidly freezing and grounding the mixed liquid to obtain a green herb extract powder; (S6) mixing the green herb extract powder with a liquid paste syrup and adding Vitamin C and food grade menthol crystal powder to produce a candy, (S7) setting and shaping the candy; and (S8) packaging the candy.

In the step (S1) of preparing nine green herbs for the first decoction to obtain the green herb extract liquid, the first-time nine natural botanical edible green herbs 1 of approximately 400 Kg are prepared, cleaned, and fine-cut, and then put into two separate metal barrels such as stainless steel barrels, where each barrel contains approximately 200 Kg of the nine natural botanical edible green herbs 1 added with approximately 300 liters of water, and heated and decocted for approximately 6-8 hours to produce approximately 100 liters of a concentrated green herb extract liquid in each metal barrel, so that the two metal barrels have a total of approximately 200 liters; the green herbs 1 of this embodiment are dried edible plant materials including fish mint (scientific name: Houttuynia Cordata Thunb) 11, cut-leaf ground berry (scientific name: Physalis Angulaa L) 12, Indian spherical turmeric (scientific name: Curcuma Longa) 13, flemingia macrophylla (common name: Root of Moghania; scientific name: Monghania Macrophylla (Willd) O Kuntze) 14, dried ginger (scientific name: Zingiber Officinale) 15, Indian peppermint (common name: Spanish thyme or Mexican mint; scientific name: Coleus Ambonicus Lour) 16, wakame (scientific name Undaria Pinnatifida) 17, Indian Echinacea (common name: Chuanxinlian; scientific name: Andrographis Paniculata (Burmf) Nees) 18 and Spreading Hedyotis (common name: Snake Needle Grass; scientific name: Hedyotis Diffusa Willd) 19; wherein the fish mint (scientific name: Houttuynia Cordata Thunb) 11 is a saururaceae; the cut-leaf ground berry (scientific name: Physalis Angulaa L) 12 is a solanaceae; the Indian spherical turmeric (scientific name: Curcuma Longa) 13 is a zingiberaceae; the flemingia macrophylla (common name: Root of Moghania; scientific name: Monghania Macrophylla (Willd) O Kuntze) 14 is a fabaceae; the dried ginger (scientific name: Zingiber Officinale) 15 is a zingiberaceae; the Indian peppermint (common name: Spanish thyme or Mexican mint; scientific name: Coleus Ambonicus Lour) 16 is a lamiaceae; the wakame (scientific name: Undaria Pinnatifida) 17 is a pterophyceae; the Indian Echinacea (common name: Chuanxinlian; scientific name: Andrographis Paniculata (Burmf) Nees) 18 is a acanthaceae; the Spreading Hedyotis (common name: Snake Needle Grass; scientific name: Hedyotis Diffusa Willd) 19 is a rubiaceae. In addition, the natural green herbs 1 of this disclosure are further processed as follows: (1) Removal of impurities: The green herbs 1 are cut to pieces of approximately 7 cm, and those shorter than 7 cm will not be cut, and then rinsed quickly without soaking, and 400±0.2kg of the rinsed green herbs 1 are divided in equal amount and put into two metal barrels respectively, where there are two metal barrels, each having an inner barrel with a capacity of 200 liters and a plurality of metal holes of 0.5cm; (2) Addition of clean water to the barrels: Each outer barrel is a stainless steel barrel and filled with 600 liters of water, and each inner barrel is filled with approximately 300 liters of water; (3) Large gas stove: Each of the two stainless steel barrels is placed on the stove, and the two stainless steel barrels are separated from each other; (4) The heating temperature of the stove is adjusted to approximately 100 °C for boiling for approximately two hours, such that the temperature of the stainless steel barrels keep boiling, and a thermometer is installed on an outer side of each stainless steel barrel, the stainless steel barrels have exhaust outlet holes, volume scales, and glass straight tubes; (5) After 6-8 hours of decoction, the first concentrated green herb extract liquid is poured out and filtered to produce a filtrate for future use.

In the step (S2), the green herb residues remained after the first decoction are used for a second decoction to obtain a green herb extract liquid. The green herb residues of the nine natural botanical edible green herbs remained after the first decoction of the step (S1) are further decocted, concentrated and extracted to obtain a green herb extract liquid. For the second time, approximately 250-300 liters are added to the green herb extract liquid, and then put into the metal barrels for heating, decoction and concentration for approximately 6-8 hours to obtain approximately 200 liters of green herb extract liquid; the green herb extract liquid obtained from the second-decoction is filtered and prepared to be used in the steps (S2), (S3), (S4), and (S5) in the same way as the step (S1).

In the step (S3), the first- and second-decoction extracted liquids are re-extracted to obtain a green herb extract concentrated liquid. The first- and second-decoction green herb extract liquids obtained from the steps (S1) and (S2) are approximately 400 liters, which are heated and concentrated to approximately 170 liters of the green herb extract concentrated liquid containing high-concentration alkaloids; the first- and second-decoction extracted liquids are poured into each stainless steel barrel. In other words, the first- and second decoction extracted liquids are combined, and a total of approximately 400 liters of filtered liquid is produced in the two decoction in each metal barrel and then mixed into a large stainless steel barrel with the capacity of approximately 600 liters, and the large stainless steel barrel is set on a large gas stove to heat the decocted liquid to a boiling temperature of approximately 100 °C ; a medium-low heat is adjusted and controlled to a temperature of approximately 100°C, the boiling steam overflow is maintained to concentrate the decocted liquid to approximately 170±0.2 liters, where the amount of liquid can be observed from the scale of the stainless steel barrel.

In the step (S4), five second-decoction plant powder granules and a small amount of excipients are added to the green herb extract concentrated liquid and mixed and stirred to obtain a mixed liquid S4. Five second-decoction plant powder granules are added to the amount of approximately 170 liters of the green herb extract concentrated liquid of the nine botanical green herbs obtained in the step (S3) at the temperature of approximately 60°C -80°C, and the second-decoction plant powder granule includes noni fruit powder 110, maca powder 111, acanthopanax powder 112, rhodiola powder 113 and naturally extracted strip menthol crystal grain 114 and corn starch and maltodextrin as excipients 119 which are mixed and stirred uniformly. There are a total of 14 green herbs, which are set still at room temperature to obtain approximately 170 liters of a mixed liquid;

In the step (S5), the mixed liquid is frozen rapidly and grounded to obtain a green herb extract powder. The amount of approximately 170 liters of the mixed liquid obtained from the step (S4) is put into a freezing equipment at -20°C±2°C to rapidly freeze the mixed liquid to a flake solid, which is grounded to obtain approximately 9.5 Kg of a green herb extract powder.

In the step (S6), the green herb extract powder is mixed with liquid paste syrup and added with Vitamin C and food grade menthol crystal powder to produce a candy. The amount of approximately 9.5 Kg of the green herb extract powder obtained from the step (S5) is mixed with approximately 390 Kg of granulated sugar 115 and maltose 116, and then heated to 60±0.2°C to dissolve and form into a liquid paste syrup (which serves as a candy base agent), and then added with Vitamin C 117 and food grade menthol crystal powder 118 (with a precise quantity) and stirred, and the temperature is maintained at 40-45°C, and the mixture is extruded into a machine tube extrusion mold to produce the candy 1000, and each piece of candy 1000 weighs approximately 4.8±0.1 grams.

In the step (S7), the candy is set still and shaped. The candy 1000 is set still for two days and cooled to room temperature.

In the step (S8), the candy is packaged. Each piece of the candy 1000 obtained from the step (S7) is wrapped with an aluminium foil 2 to produce a product. In other words, the candy 1000 of this disclosure is manufactured into a granule shape and wrapped with the aluminium foil 2 to form an independently packaged bitter tea candy 1000, and the aluminium foil 2 is used for the package of the food product by machines. The bitter tea candy of this disclosure concurrently has the cooling taste and natural bitterness and each gram of the bitter tea candy contains approximately 1.0±0.2 of green herbs which can clear the voice and soothe the throat.

In the bitter tea candy of this disclosure 1000, each piece weighs approximately 4.8±0.1 grams, and contains approximately 4.8±0.1 grams of concentrated green herb extracts. (1) Each gram of bitter tea candy contains: approximately 2.45±0.1mg/g of total alkaloid; (2) each gram of bitter tea candy contains approximately 4.13±0.1mg/g of mannitol; (3) The bitter tea candy also contains approximately 1.41±0.1mg/100g of calcium, approximately 0.6±0.1mg/100g of iron, approximately 26.2±0.1mg/100g of magnesium, approximately 0.1±0.01mg/100g of selenium; (4) The bitter tea candy also contains approximately 1.22±0.1mg/g of total polyphenol; (5) The bitter tea candy also contains approximately 10±0.1mg/g of menthol (The melting point of menthol is 44°C, and thus some of the menthol has already been vaporized), and the menthol is measured in the unit of approximately ±1.0mg; (6) The bitter tea candy 1000 has the appropriate bitterness of green herbs, the original taste of alkaloid and the cooling taste of menthol; (7) The bitter tea candy of this disclosure is in the form of a hard candy, or an oral tablet (hard mouth candy), with a pH value of 5.32±0.1, and it is the best form for slowly releasing the ingredient to soothe the discomfort of human throat (including respiratory tract), and it takes approximately 5-10 minutes for each piece of candy to dissolve in the mouth.

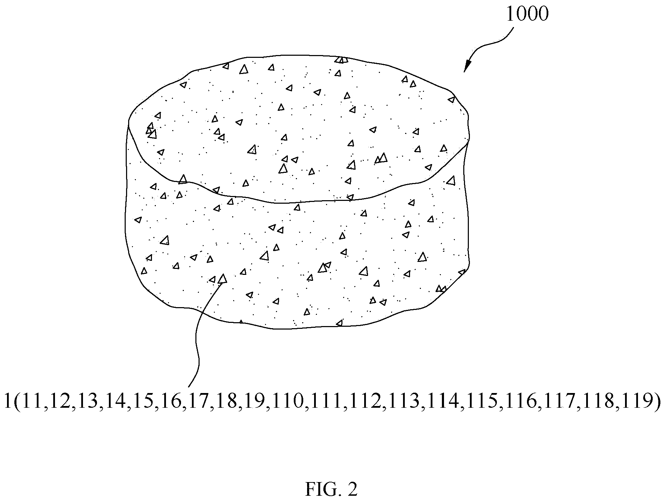

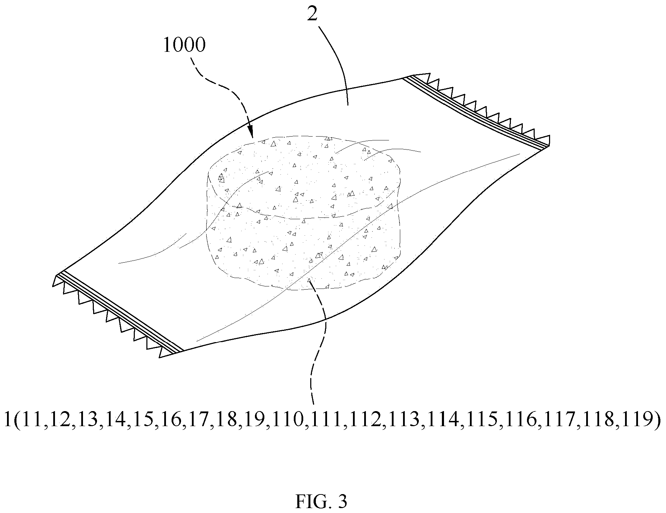

With reference to FIGS. 2 and 3 for the perspective views showing a bitter tea candy and the bitter tea candy wrapped with an aluminium foil of this disclosure respectively, the bitter tea candy 1000 is manufactured by the foregoing bitter tea candy, and the bitter tea candy 1000 includes a plurality of green herb extract powders formed by concentrating, freezing and drying a plurality of green herbs and mixed with granulated sugar 115, maltose 116, Vitamin C 117 and food grade menthol crystal powder 118 to produce the bitter tea candy 1000 in s granule shape, and each piece of the bitter tea candy 1000 weighs approximately 4.8±0.1 grams, and the bitter tea candy 1000 is wrapped with an aluminium foil 2 to prevent moisture and facilitate carrying. The green herb extract powder of the bitter tea candy of this disclosure 1000 contains: fish mint (scientific name: Houttuynia Cordata Thunb) 11, cut-leaf ground berry (scientific name: Physalis Angulaa L) 12, Indian spherical turmeric (scientific name: Curcuma Longa) 13, flemingia macrophylla (common name: Root of Moghania; scientific name: Monghania Macrophylla (Willd) O Kuntze) 14, dried ginger (scientific name: Zingiber Officinale) 15, Indian peppermint (common name: Mexican mint; scientific name: Coleus Ambonicus Lour) 16, wakame (scientific name: Undaria Pinnatifida) 17, Indian echinacea (common name: Chuanxinlian; scientific name: Andrographis Paniculata (Burmf) Nees) 18, spreading hedyotis (common name: Snake Needle Grass; scientific name: Hedyotis Diffusa Willd) 19, noni fruit powder (scientific name: Morinda Citrifolia) 110, maca powder (scientific name: Lepidm Meyenli Walp; alias: Lepidium Peruvianum) 111, acanthopanax powder (scientific name: Eleuthero Coccus Sentticosus) 112, rhodiola powder (scientific name: Rhodiola Rosea L) 113 and naturally extracted strip menthol crystal grain (scientific name: Menthol) 114 and corn starch and maltodextrin as excipients 119; each gram of the bitter tea candy 1000 contains approximately 2.45±0.1mg/g of total alkaloid, approximately 4.13±0.1mg/g of mannitol, approximately 1.41±0.1mg/100g of calcium, approximately 0.6±0.1mg/100g of iron, approximately 26.2±0.1mg/100g of magnesium, approximately 0.1±0.01mg/100g of selenium, approximately 1.22±0.1mg/g of total polyphenol, approximately menthol 10±0.1mg/g of menthol; the bitter tea candy 1000 is in a form including but not limited to a hard candy or an oral tablet with a pH value of 5.32±0.02.

The bitter tea candy manufacturing method of this disclosure includes the steps of: (S1) preparing nine green herbs for a first decoction to obtain a green herb extract liquid; (S2) using the green herb residues remained after the first decoction for a second decoction to obtain a green herb extract liquid; (S3) re-extracting the first- and second-decoction extracted liquids to obtain a green herb extract concentrated liquid; (S4) adding, mixing and stirring five second-decoction plant powder granules and a small amount of excipients in the green herb extract concentrated liquid to obtain a mixed liquid; (S5) rapidly freezing and grounding the mixed liquid to obtain a green herb extract powder; (S6) mixing the green herb extract powder with a liquid paste syrup and then adding Vitamin C and food grade menthol crystal powder to produce a candy; (S7) setting and shaping the candy; and (S8) packaging the candy. The bitter tea candy 1000 manufactured by the aforementioned bitter tea candy manufacturing method contains a plurality of green herb extract powders formed by concentrating, freezing and drying the green herbs, and the plurality of green herb extract powders is mixed with granulated sugar 115, maltose 116, Vitamin C117 and food grade menthol crystal powder 118 to produce a bitter tea candy 1000 in a granule shape, and the bitter tea candy 1000 is wrapped with an aluminium foil 2, and thus achieving a consistent manufacturing process, and the bitter tea candy in form of a hard candy or an oral tablet (hard mouth candy) has the appropriate bitterness of herb alkaloids and the cooling taste of menthol, and this disclosure significantly improves the convenience of use and carrying and increases the practical utilization in the industry. In addition, this disclosure is novel and inventive.

## Claims

1. A bitter tea candy manufacturing method, comprising the steps of:
(S1) preparing nine green herbs for a first decoction to obtain a green herb extract liquid: preparing approximately 400 Kg of first-time nine natural botanical edible green herbs, putting them into two metal barrels after cleaning and fine cutting, adding approximately 200 Kg of the nine natural botanical edible green herbs and approximately 300 liters of water into each metal barrel and heating approximately 6-8 hours for the decoction to concentrate the green herb extract liquid to approximately 100 liters in each metal barrel, wherein the two metal barrels contain approximately a total of 200 liters; and the green herb is a dried edible plant material comprising fish mint (scientific name: Houttuynia Cordata Thunb), cut-leaf ground berry (scientific name: Physalis Angulaa L), Indian spherical turmeric (scientific name: Curcuma Longa), flemingia macrophylla (common name: Root of Moghania; scientific name: Monghania Macrophylla (Willd) O Kuntze), dried ginger (scientific name: Zingiber Officinale), Indian peppermint (common name: Spanish thyme or Mexican mint; scientific name: Coleus Ambonicus Lour), wakame (scientific name Undaria Pinnatifida), Indian Echinacea (common name: Chuanxinlian; scientific name: Andrographis Paniculata (Burmf) Nees) and Spreading Hedyotis (common name: Snake Needle Grass; scientific name: Hedyotis Diffusa Willd);
(S2) using green herb residues remained after the first decoction for a second decoction to obtain a green herb extract liquid: decocting, concentrating and extracting the green herb residues remained after the first decoction of the nine natural botanical edible green herbs to obtain the green herb extract liquid in the step (S1), and adding approximately 250-300 liters of water for the second time and putting them into the metal barrels to heat, decoct and concentrate for approximately 6-8 hours to obtain approximately 200 liters of a green herb extract liquid;
(S3) re-extracting the first- and second-decoction extracted liquid to obtain a green herb extract concentrated liquid: re-extracting the first- and second-decoction green herb extract liquid to obtain approximately 400 liters of the green herb extract liquid, heating and concentrating the green herb extract liquid to approximately 170 liters of a high-concentration alkaloid green herb extract concentrated liquid;
(S4) Adding, mixing and stirring five second-decoction plant powder granules and a small amount of excipients in the green herb extract concentrated liquid to obtain a mixed liquid: adding five second-decoction plant powder granules comprising noni fruit powder, maca powder, acanthopanax powder, rhodiola powder and naturally extracted strip menthol crystal grain and corn starch and maltodextrin as excipients to approximately 170 liters of the green herb extract concentrated liquid of the nine botanical green herbs of the step (S3) at a temperature approximately 60 °C -80°C, where there are fourteen types of plants, and setting at room temperature to obtain approximately 170 liters of a mixed liquid;
(S5) rapidly freezing and grounding the mixed liquid to obtain a green herb extract powder: putting approximately 170 liters of the mixed liquid of the step (S4) into a freezing equipment at -20°C to freeze the mixed liquid to a flake solid, and grounding the flake solid to obtain approximately 9.5 Kg of a green herb extract powder;
(S6) mixing a liquid paste syrup with the green herb extract powder and then adding Vitamin C and food grade menthol crystal powder to produce a candy: mixing and heating approximately 9.5 Kg of the green herb extract powder of the step (S5) with approximately 390 Kg of granulated sugar and maltose to 60°C to form a liquid paste syrup, which is used as a candy base agent, and then adding, mixing and uniformly stirring Vitamin C and food grade menthol crystal powder in the liquid paste syrup, maintaining a temperature of 40-45°C, and extruding into a machine tube extrusion mold to produce a candy, wherein each piece of the candy weighs approximately 4.8±0.1 grams;
(S7) setting and shaping the candy: setting the candy still for two days and cooling the candy to room temperature; and
(S8) packaging the candy: wrapping each piece of candy of step (S7) with an aluminium foil to produce a product.

2. A bitter tea candy, manufactured by the bitter tea candy manufacturing method, comprising: a plurality of green herb extract powders formed by concentrating, freezing and drying a plurality of green herbs, and mixed with granulated sugar, maltose, Vitamin C and food grade menthol crystal powder to produce the bitter tea candy in a granule shape, and each piece of the bitter tea candy weighs approximately 4.8±0.1 grams.

3. The bitter tea candy according to claim 2, wherein the bitter tea candy is wrapped with an aluminium foil.

4. The bitter tea candy according to claim 2, wherein the green herb extract powder of the bitter tea candy comprises fish mint (scientific name: Houttuynia Cordata Thunb), cut-leaf ground berry (scientific name: Physalis Angulaa L), Indian spherical turmeric (scientific name: Curcuma Longa), flemingia macrophylla (common name: Root of Moghania; scientific name: Monghania Macrophylla (Willd) O Kuntze), dried ginger (scientific name: Zingiber Officinale), Indian peppermint (common name: Mexican mint; scientific name: Coleus Ambonicus Lour), wakame (scientific name: Undaria Pinnatifida), Indian Echinacea (common name: Chuanxinlian; scientific name: Andrographis Paniculata (Burmf) Nees), Spreading Hedyotis (common name: Snake Needle Grass; scientific name: Hedyotis Diffusa Willd), noni fruit powder (scientific name: Morinda Citrifolia), maca powder (scientific name: Lepidm Meyenli Walp; alias: Lepidium Peruvianum), acanthopanax powder (scientific name: Eleuthero Coccus Sentticosus), rhodiola powder (scientific name: Rhodiola Rosea L), naturally extracted strip menthol crystal grain (scientific name: Menthol), and corn starch and maltodextrin as excipients.

5. The bitter tea candy according to claim 3, wherein the green herb extract powder of the bitter tea candy comprises fish mint (scientific name: Houttuynia Cordata Thunb), cut-leaf ground berry (scientific name: Physalis Angulaa L), Indian spherical turmeric (scientific name: Curcuma Longa), flemingia macrophylla (common name: Root of Moghania; scientific name: Monghania Macrophylla (Willd) O Kuntze), dried ginger (scientific name: Zingiber Officinale), Indian peppermint (common name: Mexican mint; scientific name: Coleus Ambonicus Lour), wakame (scientific name: Undaria Pinnatifida), Indian Echinacea (common name: Chuanxinlian; scientific name: Andrographis Paniculata (Burmf) Nees), Spreading Hedyotis (common name: Snake Needle Grass; scientific name: Hedyotis Diffusa Willd), noni fruit powder (scientific name: Morinda Citrifolia), maca powder (scientific name: Lepidm Meyenli Walp, alias: Lepidium Peruvianum), acanthopanax powder (scientific name: Eleuthero Coccus Sentticosus), rhodiola powder (scientific name: Rhodiola Rosea L) and naturally extracted strip menthol crystal grain (scientific name: Menthol) and corn starch and maltodextrin as excipients.

6. The bitter tea candy according to claim 4, wherein each gram of the bitter tea candy comprises approximately 2.45±0.1mg/g of total alkaloid, approximately 4.13±0.1mg/g of mannitol, approximately 1.41±0.1mg/100g of calcium, approximately 0.6±0.1mg/100g of iron, approximately 26.2±0.1mg/100g of magnesium, approximately 0.1±0.01mg/100g of selenium, approximately 1.22±0.1mg/g of total polyphenol, and approximately 10±0.1mg/g of menthol.

7. The bitter tea candy according to claim 5, wherein each gram of the bitter tea candy comprises approximately 2.45±0.1mg/g of total alkaloid, approximately 4.13±0.1mg/g of mannitol, approximately 1.41±0.1mg/100g of calcium, approximately 0.6±0.1mg/100g of iron, approximately 26.2±0.1mg/100g of magnesium, approximately 0.1±0.01mg/100g of selenium, approximately 1.22±0.1mg/g of total polyphenol, and approximately 10±0.1mg/g of menthol.

8. The bitter tea candy according to claim 4, wherein the bitter tea candy is in a form including but not limited to a hard candy or an oral tablet, with a pH value of 5.32±0.02.

9. The bitter tea candy according to claim 5, wherein the bitter tea candy is in a form including but not limited to a hard candy or an oral tablet, with a pH value of 5.32±0.02.
